(19)

Europäisches
Patentamt
European
Patent Office
Office européen
des brevets

(11)  **EP 3 805 398 A1**

(12)  **EUROPEAN PATENT APPLICATION**
published in accordance with Art. 153(4) EPC

(43) Date of publication:
**14.04.2021 Bulletin 2021/15**

(21) Application number: **19812370.5**

(22) Date of filing: **30.05.2019**

(51) Int Cl.:
**C12P 7/04** (2006.01)    **C12M 1/00** (2006.01)

(86) International application number:
**PCT/JP2019/021450**

(87) International publication number:
**WO 2019/230861 (05.12.2019 Gazette 2019/49)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **31.05.2018 JP 2018104634**

(71) Applicant: **Toray Industries, Inc.
Tokyo, 103-8666 (JP)**

(72) Inventors:
• MIZUNO, Hiroyuki
  Kamakura-shi, Kanagawa 248-8555 (JP)
• MIMITSUKA, Takashi
  Kamakura-shi, Kanagawa 248-8555 (JP)
• SUDA, Kazumi
  Kamakura-shi, Kanagawa 248-8555 (JP)
• HIGASA, Masashi
  Kamakura-shi, Kanagawa 248-8555 (JP)

(74) Representative: **Kador & Partner PartG mbB
Corneliusstraße 15
80469 München (DE)**

(54)  **METHOD FOR PRODUCING VOLATILE CHEMICAL AND FERMENTATION DEVICE**

(57)     The present invention is a method of producing a volatile chemical substance using a culture apparatus including: a culture tank configured to culture a microorganism having an ability to produce a volatile chemical substance; a delivery line configured to deliver a culture liquid obtained by culture in the culture tank to the storage tank; and a storage tank configured to store a solution containing the chemical substance obtained by culturing the microorganism, the method including culturing the microorganism in the culture tank while retaining the solution in the storage tank, wherein a gas phase portion in the storage tank and a gas phase portion in the culture tank are connected to each other through a gas connection pipe.

[FIG. 1]

EP 3 805 398 A1

**Description**

BACKGROUND OF THE INVENTION

Field of the Invention

The present invention relates to a production method and a production apparatus, which are capable of reducing loss of a volatile chemical substance.

Background Art

Methods of producing various chemical substances with microorganisms such as yeast and bacteria, using organic compounds in biomass as fermentation feedstocks, have been practically used. In production of a chemical substance by a fermentation method, fermentation in a culture tank is followed by extraction of the chemical substance of interest from the culture liquid, or concentration of the culture liquid, in some cases. In such cases, the culture liquid is once collected into a storage tank or the like.

In cases where the chemical substance produced is a volatile chemical substance, evaporation of the volatile chemical substance from the culture liquid occurs. Therefore, the culture tank in which the fermentation is carried out, and the storage tank, are filled with a gas containing the chemical substance. Delivery of the culture liquid into the storage tank filled with the gas containing the chemical substance is dangerous since the pressure in the tank increases. Therefore, an apparatus or treatment for decreasing the pressure in the tank, such as placement of a vent for releasing the gas to the outside of the tank, is required. However, releasing of the gas to the outside of the tank also causes release of the chemical substance contained in the gas to the outside of the tank, resulting in a decrease in the chemical substance of interest collected, leading to a loss.

Patent Document 1 describes a method in which, in production of ethanol by a fermentation method, the gas in the culture tank is sucked to maintain a reduced pressure in the culture tank, to thereby reduce the ethanol concentration in the culture liquid while maintaining the fermentation rate.

In culture for a volatile chemical substance, there is a method in which a backflow condenser is placed for a vent hole for releasing the gas from the culture tank. The backflow condenser is also called cold trap. A backflow condenser placed for a culture tank or storage tank comprises a pipe through which a coolant flows, around or inside a line that connects the gas phase portion in the tank to the outside of the tank. The backflow condenser allows condensation of condensable components contained in the gas, to prevent an increase in the pressure in the tank so that loss of the volatile chemical substance can be prevented. It is thought that, by placement of backflow condensers not only for the culture tank, but also for the storage tank, loss of the volatile chemical substance can be further suppressed.

As another method of preventing loss of a volatile chemical substance, a method in which culture is performed while supplying a gas containing the volatile chemical substance of interest has been studied. For example, in Patent Document 2, loss of a volatile chemical substance in a process of methanol production is prevented by performing culture while supplying a gas containing methanol.

Prior Art Documents

[Patent Documents]

Patent Document 1: JP 2010-246512 A
Patent Document 2: JP 2005-13048 A

SUMMARY OF THE INVENTION

The present inventors studied on suppression of loss of a volatile chemical substance by placement of a backflow condenser for a storage tank, and discovered a new problem that placement of a backflow condenser for a storage tank does not enable sufficient suppression of loss of the volatile chemical substance. More specifically, even by placement of the backflow condenser, condensation of all volatile substances is difficult. The amount of substances that can be collected by condensation at least at the temperature of the cooling water is limited, and the other substances are discharged as a gas. Also in cases where the heat transfer area of the backflow condenser is insufficient, a part of the substances are discharged to cause a loss. Thus, placement of backflow condensers not only for the culture tank, but also for the storage tank, causes further dilution of the culture liquid. Moreover, although the method of Patent Document 1 enables prevention of loss of the volatile chemical substance, the method requires energy for reducing the pressure in the culture tank. The required energy increases as the size of the apparatus increases. Further, since the inside of the culture tank is controlled to be in a reduced-pressure state, the concentration of the chemical substance contained in the culture liquid also decreases.

An object of the present invention is to provide an apparatus and a method which simply prevent loss of a volatile chemical substance from a storage tank, without using a backflow condenser for the storage tank.

As a result of intensive study, the present inventors discovered that, by connecting a gas phase portion in a fermentation tank and a gas phase portion in a storage tank to each other through a gas connection pipe, loss of a volatile chemical substance from the storage tank can be simply reduced, thereby accomplishing the present invention.

More specifically, the present invention is as described below in [1] to [9].

[1] A method of producing a volatile chemical substance using a culture apparatus comprising: a culture tank configured to culture a microorganism having an ability to produce a volatile chemical substance; a storage tank configured to store a solution containing the chemical substance obtained by culturing the microorganism; and a delivery line configured to deliver a culture liquid obtained by culturing in the culture tank to the storage tank,
the method comprising culturing the microorganism in the culture tank while retaining the solution in the storage tank,
wherein a gas phase portion in the storage tank and a gas phase portion in the culture tank are connected to each other through a gas connection pipe.
[2] The method of producing a volatile chemical substance according to [1],
wherein the delivery line comprises: a separation membrane module configured to separate microorganisms in the culture liquid; a culture liquid delivery line configured to deliver the culture liquid to the separation membrane module; a filtrate pipe configured to deliver a filtrate obtained by the separation membrane module to the storage tank; and an unfiltered -liquid pipe configured to return an unfiltered liquid obtained by the separation membrane module to the culture tank, and
wherein in the delivery of the culture liquid from the culture tank to the storage tank, a filtrate obtained by passing the culture liquid through the separation membrane module is delivered to the storage tank as a solution containing the volatile chemical substance, and an unfiltered liquid obtained by passing the culture liquid through the separation membrane module is returned to the culture tank.
[3] The method of producing a volatile chemical substance according to [2], wherein the separation membrane has an average pore size of 0.01 $\mu$m or more and less than 5 $\mu$m.
[4] The method of producing a volatile chemical substance according to [2] or [3], wherein the separation membrane has a transmembrane pressure of 0.1 kPa or more and 150 kPa or less.
[5] The method of producing a volatile chemical substance according to any one of [1] to [4], wherein the liquid temperature in the culture tank is higher than the liquid temperature in the storage tank.
[6] The method of producing a volatile chemical substance according to any one of [1] to [5], wherein the vapor pressure of the volatile chemical substance is 1 kPa or more and 101 kPa or less at 30°C.
[7] The method of producing a volatile chemical substance according to any one of [1] to [6], wherein the volatile chemical substance is ethanol, methanol, 1-propanol, 2-propanol, 1-butanol, 2-methyl-1-propanol, 2-butanol, 2-methyl-2-propanol, acetone, or acetic acid.
[8] An apparatus for producing a volatile chemical substance, the apparatus comprising: a culture tank configured to culture a microorganism having an ability to produce a volatile chemical substance; a storage tank configured to store a solution containing the chemical substance obtained by culturing the microorganism; a delivery line configured to deliver a culture liquid obtained by culturing in the culture tank to the storage tank; and a gas connection pipe configured to connect a gas phase portion in the culture tank and a gas phase portion in the storage tank to each other.
[9] The apparatus for producing a volatile chemical substance according to [8], wherein the delivery line comprises: a separation membrane module configured to separate microorganisms in the culture liquid; a culture liquid delivery line configured to deliver the culture liquid to the separation membrane module; a filtrate pipe configured to deliver a filtrate obtained by the separation membrane module to the storage tank as a solution containing the volatile chemical substance; and an unfiltered-liquid pipe configured to return an unfiltered liquid obtained by the separation membrane module to the culture tank.

According to the present invention, the gas generated in the storage tank moves to the fermentation tank through the gas connection pipe without being discharged to the outside, so that loss of a volatile chemical substance in the fermentation tank and the storage tank can be simply reduced.

BRIEF DESCRIPTION OF THE DRAWINGS

FIG. 1 is a schematic diagram for illustration of a culture apparatus of the present invention comprising a culture tank, a storage tank, a delivery line, and a gas connection pipe.

FIG. 2 is a schematic diagram for illustration of a culture apparatus of the present invention comprising a multi-stage culture tank, a storage tank, a delivery line, and a gas connection pipe.

FIG. 3 is a schematic diagram for illustration of a culture apparatus of the present invention comprising a continuous-culture tank, a storage tank, a delivery line, and a gas connection pipe.

FIG. 4 is a schematic diagram for illustration of the culture apparatus used in Example 1, which comprises a gas connection pipe.

FIG. 5 is a schematic diagram for illustration of the culture apparatus used in Comparative Example 1, which comprises no gas connection pipe.

FIG. 6 is a schematic diagram for illustration of the culture apparatus used in Comparative Example 2, which comprises no gas connection pipe.

FIG. 7 is a schematic diagram for illustration of the continuous-culture apparatus used in Example 2, which comprises a gas connection pipe.

FIG. 8 is a schematic diagram for illustration of the continuous-culture apparatus used in Comparative Example 3, which comprises no gas connection pipe.

FIG. 9 is a schematic diagram for illustration of the continuous-culture apparatus used in Comparative Example 4, which comprises no gas connection pipe.

FIG. 10 is a diagram presenting the ethanol concentrations in the storage tanks in Example 1, and Comparative Examples 1 and 2.

## DETAILED DESCRIPTION OF THE INVENTION

The present invention is a method of producing a volatile chemical substance using a culture apparatus comprising: a culture tank configured to culture a microorganism having an ability to produce a volatile chemical substance; a storage tank configured to store a solution containing the chemical substance obtained by culturing the microorganism; and a delivery line configured to deliver a culture liquid obtained by culturing in the culture tank to the storage tank, the method comprising culturing the microorganism in the culture tank while retaining the solution in the storage tank, and is technically characterized in that a gas phase portion in the storage tank and a gas phase portion in the culture tank are connected to each other through a gas connection pipe. The gas connection pipe may be connected to any portions of the culture tank and the storage tank as long as the liquids in these tanks are not mixed together. The shape of the gas connection pipe is not limited as long as the pipe has a tubular shape. The material of the gas connection pipe is preferably a metal from the viewpoint of durability. However, the material may also be a resin. The inner diameter of the gas connection pipe is not limited as long as the pipe has a size that easily allows movement of a gas.

According to the method of the present invention, the gas phase portion in the storage tank storing the solution containing the volatile chemical substance is connected to the gas phase portion in the culture tank retaining the culture liquid, through the gas connection pipe. Therefore, due to the difference between the pressure in the storage tank and the pressure in the culture tank, the gas in the storage tank and the gas in the culture tank can move in both directions such that the pressure becomes uniform, or can move in both directions such that the concentration of the volatile chemical substance becomes uniform between the gas phase in the storage tank and the gas phase in the culture tank, to suppress loss of the volatile chemical substance. More specifically, compared to cases without the connection through the gas connection pipe, the concentration of the volatile chemical substance in the solution containing the volatile chemical substance, stored in the storage tank, can be kept high. The connection through the gas connection pipe may be either permanent connection in the configuration of the culture apparatus, or temporary connection.

The culture tank retaining the culture liquid may be either in a state during culture or in a state after completion of culture.

The solution containing the volatile chemical substance corresponds to the culture liquid retained in the culture tank, a solution after removal of microorganisms from the culture liquid, or the like.

Methods of culturing a microorganism can be roughly classified into: (1) a batch culture method or fed-batch culture method, and (2) a continuous culture method. The batch culture method or fed-batch culture method described in (1) has the effects that only simple equipment is required, that the culture can be completed in a short time, and that the influence of bacterial contamination is small. However, the concentration of the fermentation product in the culture liquid increases with time, and thus fermentation is inhibited by, for example, an increase in the osmotic pressure or the product itself, leading to a decrease in the productivity or yield. The continuous culture method described in (2) can maintain high yield and productivity of the chemical substance for a long time by continuously supplying or discharging a feedstock or fermentation liquid in order to avoid accumulation of a fermentation product at high concentration in the culture tank.

According to the method of the present invention, a loss-reducing effect on a volatile chemical substance can be expected when the volatile chemical substance is produced either by the batch culture method or fed-batch culture method described in (1), or by the continuous culture method described in (2). However, the continuous culture method described in (2) is preferred.

In the method of the present invention, the gas phase portion in the culture tank and the gas phase portion in the storage tank are connected to each other through the gas connection pipe, and culture is performed in the culture tank while retaining a solution containing the volatile chemical substance in the storage tank. As the length of time of the culture in the culture tank increases, the loss-reducing effect on the volatile chemical substance can be more remarkably expected. Regarding specific examples of the culture period, the culture period is preferably 100 hours or more, more preferably 150 hours or more, still more preferably 200 hours or more.

In cases where the culture method in the culture tank is the batch culture method or fed-batch culture method describe in (1), culture is performed in the culture tank according to this culture method, and, after completion of the culture, the culture liquid is delivered to the storage tank through the delivery line, followed by storing the culture liquid in the storage tank as a solution containing the volatile chemical substance. In this step, a part of the culture liquid may be left in the culture tank, or new culture may be begun in the culture tank. Also in the step of delivering the culture liquid through the delivery line, the gas phase portion in the culture tank and the gas phase portion in the storage tank are preferably connected to each other through the gas connection pipe.

In cases where the culture method in the culture tank is the continuous culture method described in (2), a multi-stage culture method or membrane-utilizing culture method is preferably applied. A membrane-utilizing culture method is more preferably applied. The membrane-utilizing culture method is a continuous culture method such as the method exemplified in WO 2007/097260. The method can be applied to the present invention by arranging, on the delivery line, at least a separation membrane module configured to separate microorganisms in the culture liquid, a culture liquid delivery line configured to deliver the culture liquid to the separation membrane module, a filtrate pipe configured to deliver a filtrate obtained by the separation membrane module to the storage tank, and an unfiltered-liquid pipe configured to return an unfiltered liquid obtained by the separation membrane module to the culture tank, and performing culture in the culture tank and continuous membrane filtration of the culture liquid by the method described in WO 2007/097260 while storing the resulting filtrate in the storage tank as a solution containing the volatile chemical substance. In the case of the membrane-utilizing culture method, the culture liquid is continuously delivered from the culture tank to the storage tank. Therefore, the connection between the gas phase portion in the culture tank and the gas phase portion in the storage tank through the gas connection pipe is preferably permanent connection in the configuration the culture apparatus.

The separation membrane may be either an inorganic membrane or an organic membrane as long as the separation of the microorganism from the culture liquid is possible therewith. Specific examples of the separation membrane include porous ceramic membranes, porous glass membranes, porous organic polymer membranes, metal fiber textiles, and non-woven fabrics. Among these, porous organic polymer membranes and ceramic membranes are especially preferred. For allowing sterilization of the separation membrane module by treatment of the separation membrane in saturated water vapor at 2 atm at 121°C for 20 minutes, the separation membrane preferably has heat resistance. Regarding the configuration of the separation membrane, the separation membrane preferably contains a porous resin layer as a functional layer from the viewpoint of resistance to dirt.

The separation membrane containing a porous resin layer preferably has a porous resin layer that acts as a separation function layer, on a surface of a porous substrate. The porous substrate supports the porous resin layer to give strength to the separation membrane. In cases where the separation membrane has the porous resin layer on the surface of the porous substrate, the porous substrate may be impregnated with the porous resin layer, or may not be impregnated with the porous resin layer.

The porous substrate preferably has an average thickness of 50 $\mu$m or more and 3000 $\mu$m or less. The porous substrate is composed of an organic material, inorganic material, and/or the like. In particular, an organic fiber is preferably used. Preferred examples of the organic fiber used for the porous substrate include cellulose fibers, cellulose triacetate fibers, polyester fibers, polypropylene fibers, and polyethylene fibers. Woven fabrics and non-woven fabrics of these fibers are preferably used. Non-woven fabrics are especially preferred since their densities can be relatively easily controlled, and since they can be easily and inexpensively produced.

As the porous resin layer, an organic polymer membrane may be preferably used. Examples of the material of the organic polymer membrane include polyethylene resins, polypropylene resins, polyvinyl chloride resins, polyvinylidene fluoride resins, polysulfone resins, polyethersulfone resins, polyacrylonitrile resins, cellulose resins, and cellulose triacetate resins. The organic polymer membrane may also be a resin mixture containing these resins as major components. Here, a major component means that the component is contained in an amount of 50% or more by weight, preferably 60% or more by weight. The material of the organic polymer membrane is preferably a polyvinyl chloride resin, polyvinylidene fluoride resin, polysulfone resin, polyethersulfone resin, or polyacrylonitrile resin from the viewpoint of easy formation of the membrane using a solution, and good physical durability and chemical resistance. A polyvinylidene fluoride resin or a resin containing it as a major component is most preferably used.

Here, as the polyvinylidene fluoride resin, a homopolymer of vinylidene fluoride is preferably used. The polyvinylidene fluoride resin is also preferably a copolymer with vinyl monomers capable of copolymerizing with vinylidene fluoride. Examples of the vinyl monomers capable of copolymerizing with vinylidene fluoride include tetrafluoroethylene, hexafluoropropylene, and fluoroethylene trichloride.

The pore size of the separation membrane used in the present invention is not limited as long as it does not allow passing of the microorganism used in the culture. The pore size is preferably within a range in which clogging with secretions of the microorganism used in the culture, or with fine particles in the fermentation feedstock, is less likely to occur, and in which the filtration performance can be stably maintained for a long time. Thus, the average pore size of the porous separation membrane is preferably 0.01 $\mu$m or more and 5 $\mu$m or less. The average pore size of the separation membrane is more preferably 0.01 $\mu$m or more and 1 $\mu$m or less since such an average pore size enables achievement of both a high rejection rate which does not allow leakage of the microorganism, and a high permeability, and also enables maintenance of the permeability for a long time.

From the viewpoint of preventing leakage of the microorganism, that is, preventing a trouble causing a decrease in the rejection rate, the average pore size of the separation membrane is preferably not too large relative to the size of the microorganism. In cases where a small cell such as a bacterium is used as the microorganism, the average pore size is preferably 0.01 $\mu$m or more and 0.4 $\mu$m or less, more preferably 0.01 $\mu$m or more and 0.2 $\mu$m or less.

Here, the average pore size can be determined by measuring the diameters of all pores which can be observed within an area of 9.2 $\mu$m $\times$ 10.4 $\mu$m under a scanning electron microscope at a magnification of 10,000$\times$, and averaging the measured values. Alternatively, the average pore size can be determined by taking a photograph of the membrane surface using a scanning electron microscope at a magnification of 10,000$\times$, and randomly selecting 10 pores or more, preferably 20 pores or more, followed by measuring the diameters of these pores and calculating the arithmetic average. In cases where a pore is not circular, a circle having the same area as that of the pore (equivalent circle) may be determined using an image processing device or the like, and the diameter of the equivalent circle may be regarded as the diameter of the pore.

The standard deviation $\sigma$ of the average pore size of the separation membrane used in the present invention is preferably 0.1 $\mu$m or less. It is preferable that the standard deviation $\sigma$ of the average pore size is as small as possible. The standard deviation $\sigma$ of the average pore size is calculated according to the following Equation 1, wherein N represents the number of pores observable within the above-mentioned area of 9.2 $\mu$m $\times$ 10.4 $\mu$m; Xk represents the respective measured diameters; and X(ave) represents the average of the pore sizes.

$$\sigma = \sqrt{\frac{\sum_{k=1}^{N}\left(X_k - X(ave)\right)^2}{N}} \quad \cdots \cdots \text{(Equation 1)}$$

In the separation membrane used in the present invention, permeability to the culture liquid is an important property. As an index of permeability of the separation membrane, the pure water permeability coefficient of the separation membrane before use can be used. In the present invention, the pure water permeability coefficient of the separation membrane is preferably $5.6 \times 10^{-10}$ m$^3$/m$^2$/s/pa or more as calculated by measuring the amount of permeation with a head height of 1 m using purified water at a temperature of 25°C prepared with a reverse osmosis membrane. In cases where the pure water permeability coefficient is $5.6 \times 10^{-10}$ m$^3$/m$^2$/s/pa or more and $6 \times 10^{-7}$ m$^3$/m$^2$/s/pa or less, a practically sufficient amount of permeation can be obtained.

The membrane surface roughness of the separation membrane used in the present invention is the average height in the vertical direction from the surface. The membrane surface roughness is a factor related to easiness of detachment of the microorganism adhering to the surface of the separation membrane, by the membrane surface washing effect of a liquid current produced by stirring or a circulating pump. The surface roughness of the separation membrane is not limited as long as the microorganism and other solids adhering to the membrane can be detached. The surface roughness is preferably 0.1 $\mu$m or less. In cases where the surface roughness is 0.1 $\mu$m or less, the microorganism and other solids adhering to the membrane can be easily detached.

The separation membrane more preferably has a surface roughness of 0.1 $\mu$m or less, an average pore size of 0.01 $\mu$m or more and 1 $\mu$m or less, and a pure water permeability coefficient of $2 \times 10^{-9}$ m$^3$/m$^2$/s/pa or more. It was found that, by using such a separation membrane, the operation can be more easily carried out without requiring excessive power for washing the membrane surface. In cases where the membrane surface roughness is 0.1 $\mu$m or less, the shear force generated on the membrane surface during the filtration of the microorganism can be reduced, and destruction of the microorganism can therefore be suppressed, so that clogging of the separation membrane can be suppressed. Thus, stable filtration can be more easily carried out for a long time. In cases where the membrane surface roughness of the separation membrane is 0.1 μm or less, continuous fermentation can be carried out with a smaller transmembrane pressure, and, even when clogging of the separation membrane occurs, the membrane can be more easily recovered by washing compared to cases where the operation is carried out with a large transmembrane pressure. Since stable continuous fermentation is possible by suppressing clogging of the separation membrane, it is preferable that the surface roughness of the separation membrane is as small as possible.

Here, the membrane surface roughness of the separation membrane is a value measured using the following atomic force microscope (AFM) under the following conditions. Apparatus: atomic force microscope apparatus ("Nanoscope (registered trademark) IIIa", manufactured by Digital Instruments) Measurement conditions:

Probe: SiN cantilever (manufactured by Digital Instruments)
Scanning mode: Contact mode (measurement in air); or Underwater tapping mode (underwater measurement)
Scanning area: 10 μm × 25 μm (measurement in air); or 5 μm × 10 μm (underwater measurement)
Scanning resolution: 512 × 512

For the measurement, the membrane sample was soaked in ethanol at room temperature for 15 minutes, and then soaked in RO water for 24 hours, followed by washing, and then drying in air. The RO water means water prepared by filtration through a reverse osmosis membrane (RO membrane), which is a filtration membrane, to remove impurities such as ions and salts. The pore size of the RO membrane is about 2 nm or less.

The membrane surface roughness, $d_{rough}$, is calculated according to the following Equation 2 using the above atomic force microscope apparatus (AFM), based on the height of each point in the direction of the z-axis.

$$d_{rough} = \sum_{n=1}^{N} \frac{|Z_n - \overline{Z}|}{N} \quad \cdots \text{(Equation 2)}$$

$d_{rough}$ : Surface roughness (μm)
$Z_n$ : Height in Z-axis direction (μm) : Average height in Scanned area (μm)
$N$ : Number of samples measured

The shape of the separation membrane used in the present invention is not limited, and the separation membrane may be a flat membrane, hollow fiber membrane, or the like. The separation membrane is preferably a hollow fiber membrane. In cases where the separation membrane is a hollow fiber membrane, the inner diameter of the hollow fiber is preferably 200 μm or more and 5000 μm or less, and the membrane thickness is preferably 20 μm or more and 2000 μm or less. A textile or knit produced by forming organic fibers or inorganic fibers into a cylindrical shape may be contained in the hollow fiber.

The separation membrane mentioned above can be produced by, for example, the production method described in WO 2007/097260.

The filtration through the separation membrane is controlled using the transmembrane pressure. The transmembrane pressure during the filtration is not limited as long as the culture liquid can be filtered. In cases where the transmembrane pressure is too large, the structure of the separation membrane is destroyed, while in cases where the transmembrane pressure is too small, the filtration cannot be sufficiently carried out. Thus, the transmembrane pressure is preferably within the range of 0.1 kPa or more and 150 kPa or less, more preferably within the range of 0.1 kPa or more and 50 kPa or less, most preferably within the range of 0.1 kPa or more and 20 kPa or less.

Further, in the present invention, the temperature in the storage tank is preferably controlled to a lower temperature than in the culture tank, irrespective of the culture method. By controlling the temperature in the storage tank to a lower temperature than in the culture tank, the concentration of the volatile chemical substance in the gas phase in the storage tank can be made lower than in the gas phase in the culture tank. Therefore, the volatile chemical substance moves from the culture tank toward the storage tank through the gas connection pipe by diffusion. Thus, the temperature in the storage tank storing the solution containing the volatile chemical substance is preferably lower. In the present invention, the temperature in the culture tank means the liquid temperature of the culture liquid, and the temperature in the storage tank means the liquid temperature in the storage tank.

The volatile chemical substance produced by the method of the present invention is not limited as long as it has a vapor pressure at room temperature and atmospheric pressure. The volatile chemical substance is preferably a substance which exists mainly as a liquid at room temperature and atmospheric pressure. More specifically, the volatile chemical substance is preferably a substance having a vapor pressure of 1 kPa or more and 101 kPa or less at 30°C.

Specific examples of the substance having a vapor pressure of 1 kPa or more and 101 kPa or less at 30°C include ethanol, methanol, 1-propanol, 2-propanol, 1-butanol, 2-methyl-1-propanol, 2-butanol, 2-methyl-2-propanol, acetone, and acetic acid.

In cases where generation of a gas(es) other than the volatile chemical substance of interest occurs, the volatile chemical substance is preferably produced in a culture tank containing a vent from the viewpoint of safety. In particular, in culture that generates carbon dioxide, discharge of the chemical substance of interest to the outside of the culture tank together with carbon dioxide can be suppressed by placement of a backflow condenser for the vent hole.

The microorganism used in the present invention is not limited as long as it is a microorganism having an ability to produce the volatile chemical substance. Preferred specific examples of the microorganism include yeasts such as baker's yeast; bacteria such as *E. coli* and coryneform bacteria; filamentous fungi; and actinomycetes; which are commonly used in the fermentation industry. The microorganism may be a microorganism isolated from the natural environment, or may be a microorganism whose properties are partially modified by mutation or genetic recombination.

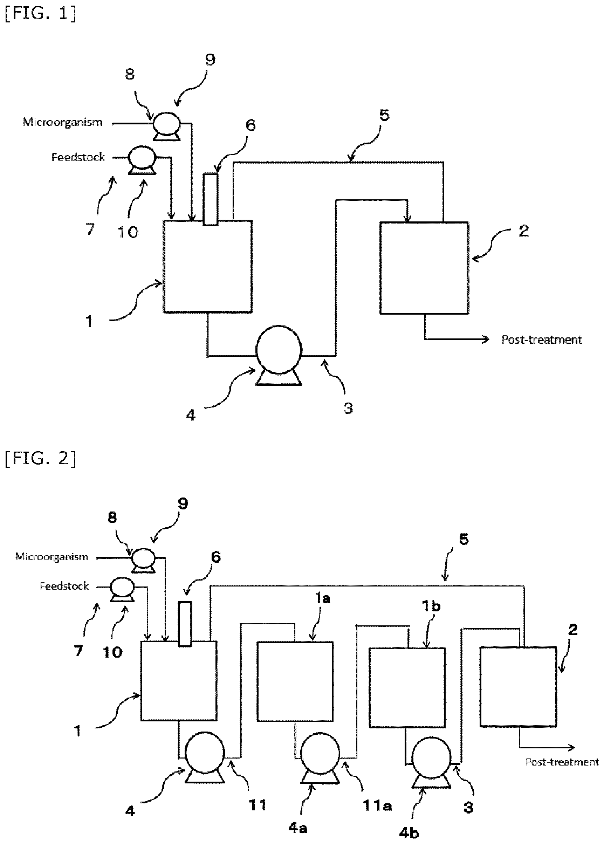

One aspect of the culture apparatus of the present invention is illustrated in FIG. 1 for a case where the culture method in the culture tank is the batch culture method or fed-batch culture method described in (1). The culture apparatus in FIG. 1 comprises: a culture tank 1 configured to culture a microorganism having an ability to produce a volatile chemical substance; a storage tank 2 configured to store a culture liquid after culture; a delivery line 3 configured to deliver the culture liquid from the culture tank 1 to the storage tank 2; and a gas connection pipe5 configured to connect a gas phase portion in the culture tank and a gas phase portion in the storage tank to each other.

In FIG. 1, the delivery line 3 includes a pump 4 as liquid delivery means, and the culture tank 1 additionally includes a feedstock supply line 7, a microorganism supply line 8, and the like. These units are not indispensable, and do not necessarily need to be included. The culture tank 1 preferably includes a vent 6 for safety. In addition to these units, the apparatus may further include a stirrer, a gas-feeding unit, a pH sensor, a pH controller, a temperature controller, a liquid level sensor, or the like, although these are not indispensable.

In the culture apparatus of FIG. 1, the culture liquid in the culture tank 1 is retained in the culture tank after completion of the fermentation, or delivered to the storage tank 2 through the delivery line 3. Since the culture tank 1 and the storage tank 2 are connected to each other through the gas connection pipe 5, the gas in the culture tank 1 and the gas in the storage tank 2 can move in both directions through the gas connection pipe 5.

When the culture liquid is delivered from the culture tank 1 to the storage tank 2, the liquid level in the culture tank 1 decreases, and the liquid level in the storage tank 2 increases, in the presence of the connection through the gas connection pipe 5. Upon the increase in the liquid level in the storage tank 2, the gas in the storage tank 2 moves toward the culture tank 1 through the gas connection pipe 5. Thus, by providing the gas connection pipe 5, discharge of the gas to the outside of the culture tank 1 and the storage tank 2, and inflow of a gas from the outside of the tanks, can be prevented.

One aspect of the culture apparatus of the present invention is illustrated in FIG. 2 for a case where the culture method in the culture tank is a multi-stage continuous culture method, which is included in the continuous culture method described in (2). Multi-stage continuous culture is a culture method using a plurality of culture tanks connected to each other. The culture tanks are connected linearly or in parallel. FIG. 2 presents an example in which three culture tanks are linearly connected to each other. The culture apparatus in FIG. 2 comprises: culture tanks 1, 1a, and 1b configured to culture a microorganism having an ability to produce a volatile chemical substance; multi-stage delivery lines 11 and 11a configured to connect the culture tanks to each other; a delivery line 3 configured to deliver the culture liquid from the culture tank 1b to the storage tank 2; a storage tank 2 configured to store the culture liquid after the culture; and a gas connection pipe 5 configured to connect a gas phase portion in the culture tank 1 and a gas phase portion in the storage tank 2 to each other.

The culture liquids in the culture tanks 1 and 1a are delivered to the culture tanks 1a and 1b, respectively, through the multi-stage delivery lines 11 and 11a.

Although FIG. 2 includes pumps 4, 4a, and 4b as liquid delivery means for the delivery line 3 and the multi-stage delivery lines 11 and 11a, these units are not indispensable, and do not necessarily need to be included in the apparatus.

When the culture liquid is delivered between the culture tanks and the storage tank 2, the liquid levels in the culture tanks and the storage tank 2 increase or decrease in the presence of the connection through the gas connection pipe 5. Since the gas in the culture tank 1 and the storage tank 2 can move through the gas connection pipe 5 in accordance with the increase or decrease in the liquid levels, discharge of the gas from the culture tank and the storage tank, and inflow of a gas from the outside of the tanks, can be prevented also in the multi-stage continuous culture method by providing the gas connection pipe.

Although the culture tanks 1, 1a, and 1b in FIG. 2 additionally include a feedstock supply line 7 and a microorganism supply line 8, these units are not indispensable, and do not necessarily need to be included. The culture tanks 1, 1a, and 1b preferably include a vent 6 for safety. In addition to these units, if necessary, the apparatus may further include a stirrer, a gas-feeding unit, a pH sensor/controller, a temperature controller, a liquid level sensor, or the like, although these are not indispensable units.

The gas phase portions in the culture tanks 1, 1a, and 1b, and in the storage tank 2, may be connected to each other through gas connection pipes.

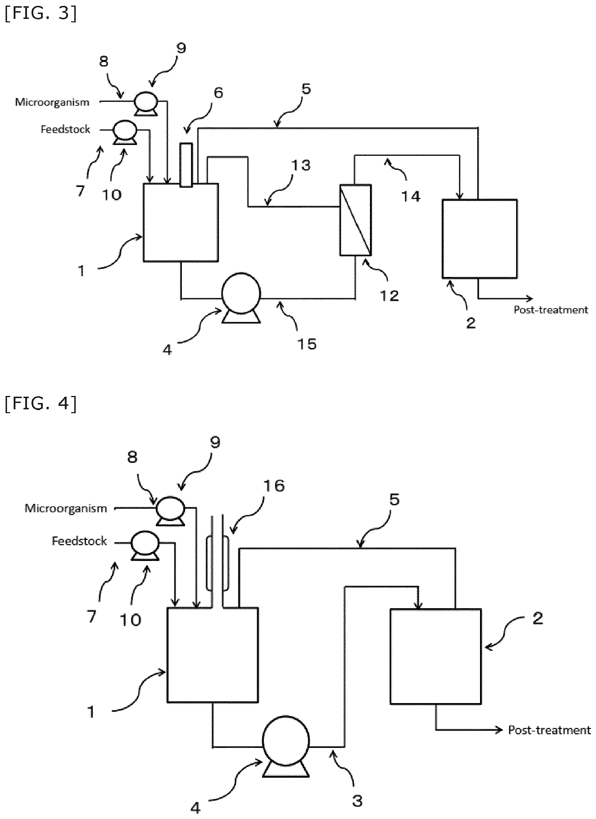

One aspect of the present culture apparatus is illustrated in FIG. 3 for a case where the culture method in the culture tank is continuous culture utilizing a separation membrane module, which is included in the continuous culture method described in (2). In the continuous culture utilizing a separation membrane module, a volatile compound is obtained by performing culture in a culture tank, and the resulting culture liquid is filtered through a separation membrane module to achieve separation between microorganisms and the filtrate. The filtrate is collected into a storage tank, while the unfiltered liquid is retained, or returned to the culture tank, and a fermentation feedstock is added to the culture tank.

The culture apparatus illustrated in FIG. 3 comprises: a culture tank 1 configured to carry out culture of a microorganism having an ability to produce a volatile chemical substance; and a separation membrane module 12 configured to filter a culture liquid. The culture tank 1 is connected to the separation membrane module 12 through a culture liquid delivery line 15 and an unfiltered-liquid pipe 13 (circulation line). The secondary side (filtrate side) of the separation membrane module 12 is connected to a storage tank 2 through a filtrate pipe 14. The gas phase portion in the storage tank 2 and the gas phase portion in the culture tank 1 are connected to each other through a gas connection pipe 5.

To the culture tank 1, a feedstock supply line 7 configured to supply a fermentation feedstock to the culture tank is connected. Although the culture tank 1 in FIG. 3 includes a microorganism supply line 8, this is not an indispensable unit, and does not necessarily need to be included. The culture tank 1 preferably includes a vent 6 for safety. In addition to these units, the apparatus may further include a stirrer, a gas-feeding unit, a pH sensor, a pH controller, a temperature controller, a liquid level sensor, or the like, although these are not indispensable units.

Although the culture liquid delivery line 15 in FIG. 3 includes a pump 4 configured to deliver the culture liquid, this is not an indispensable unit, and does not necessarily need to be included. The unfiltered-liquid pipe 13 may also include a pump, if necessary, although this is also not an indispensable unit. For control of the filtration rate, the filtrate pipe 14 in the secondary side may include a pump, control valve, or the like.

The filtrate obtained by the filtration of the culture liquid through the separation membrane module 12 is delivered to the storage tank 2 through the filtrate pipe 14. The filtrate is stored in the storage tank 2 for a certain period, and, if necessary, delivered to a post-treatment such as distillation.

The shape of the separation membrane module 12 is not limited as long as the module has a structure which enables connection of the culture liquid delivery line 15 and the unfiltered-liquid pipe 13 to the primary side, and connection of the filtrate pipe 14 to the secondary side.

In continuous culture with the culture apparatus illustrated in FIG. 3, when the culture liquid in the culture tank 1 is delivered to the separation membrane module 12 through the delivery line 15, the liquid level in the culture tank decreases by the amount of the culture liquid delivered, and the liquid level in the storage tank 2 increases by the amount of the filtrate delivered from the filtrate pipe 14. Upon the elevation of the liquid level in the storage tank 2, the gas in the storage tank moves to the culture tank 1 through the gas connection pipe 5. The culture tank 1 can receive the gas in the amount corresponding to the decrease in the liquid level.

When the liquid level in the culture tank 1 increases due to, for example, addition of a fermentation medium to the culture tank, there is no sufficient space to receive the gas moving from the storage tank. In this case, the filtrate in the storage tank may be drawn to control the liquid levels in the culture tank 1 and the storage tank 2. When the filtrate in the storage tank is collected, loss of the volatile chemical substance can be further reduced by collecting only the culture liquid by, for example, collection of the culture liquid from the bottom of the storage tank.

EXAMPLES

The present invention is described below in more detail by way of Examples and Comparative Examples. The experiments in all Examples and Comparative Examples were carried out in the state where the gas connection pipe, vent, and backflow condenser in the culture apparatus are constantly open.

(Reference Example 1) Measurement of Ethanol

The ethanol concentration was measured under the following gas chromatography conditions based on comparison with a standard sample.

Apparatus: SHIMADZU GAS CHROMATOGRAPH GC-2010
(SHIMADZU)
Column: TC-BOND Q 0.25 mm $\times$ 30 mm
Mobile phase: helium
Detection method: FID

Flow rate: 40 cm/s
Temperature: 250°C

(Reference Example 2) Measurement of Sugars

Sugar concentrations were measured under the following HPLC conditions based on comparison with standard samples.

Apparatus: SHIMADZU HPLC System (Shimadzu)
Column: HILICpak VG-50 4E (Shodex)
Mobile phase: acetonitrile:water=3:1
Detection method: RI detector
Flow rate, mobile phase: 0.6 ml/min
Temperature: 40°C

(Reference Example 3) Preparation of Molasses

Molasses and water were mixed together at a weight ratio of 1:3, to provide a fermentation feedstock. The results of analysis of sugars contained in the molasses by the method described in Reference Example 2 are shown in Table 1.

[Table 1]

|  | Glucose (g/L) | Fructose (g/L) | Sucrose (g/L) |
|---|---|---|---|
| Fermentation feedstock | 22.2 | 29.5 | 104.6 |

(Example 1)

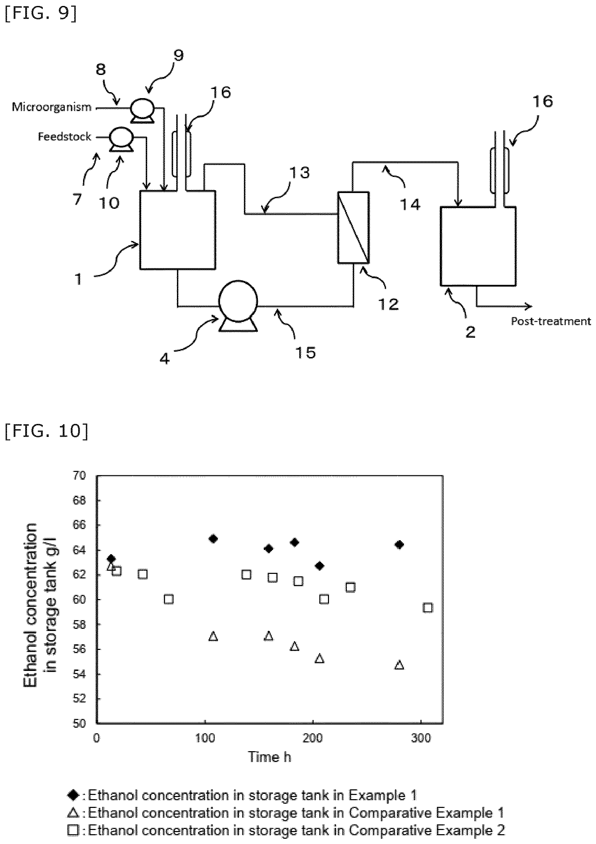

A test was carried out using the culture apparatus illustrated in FIG. 4. The *Schizosaccharomyces pombe* NBRC1628 strain was inoculated to a test tube containing 5 ml of SD medium. Culture was performed overnight with shaking (pre-preculture). The resulting culture liquid was inoculated to an Erlenmeyer flask containing 45 ml of the molasses prepared in Reference Example 3, and shake culture was performed at 30°C at 120 rpm for 8 hours (preculture). To a culture tank containing 700 ml of the molasses prepared in Reference Example 3, 35 ml out of 50 ml of the preculture liquid was inoculated, and first ethanol fermentation was carried out with stirring at 300 rpm using a stirrer with neither aeration nor neutralization. Since carbon dioxide is generated during the ethanol fermentation, a backflow condenser 16 was placed for the culture tank 1. For judging completion of the fermentation, the method in Reference Example 2 was carried out to confirm that the sugars contained in the medium have been entirely consumed. The culture liquid was delivered, through the delivery line 3, to the storage tank 2, which is connected to the culture tank 1 through the gas connection pipe 5, and then the culture liquid was retained in a state where the storage tank stores the culture liquid. Subsequently, second ethanol fermentation was carried out. The second ethanol fermentation was carried out under the same conditions as in the first ethanol fermentation, and the culture liquid after completion of the fermentation was retained as it is in the culture tank. The ethanol concentration in the storage tank was measured over time from the beginning of the second ethanol fermentation until Hour 320 by the method in Reference Example 1. The results are shown in FIG. 10.

(Comparative Example 1)

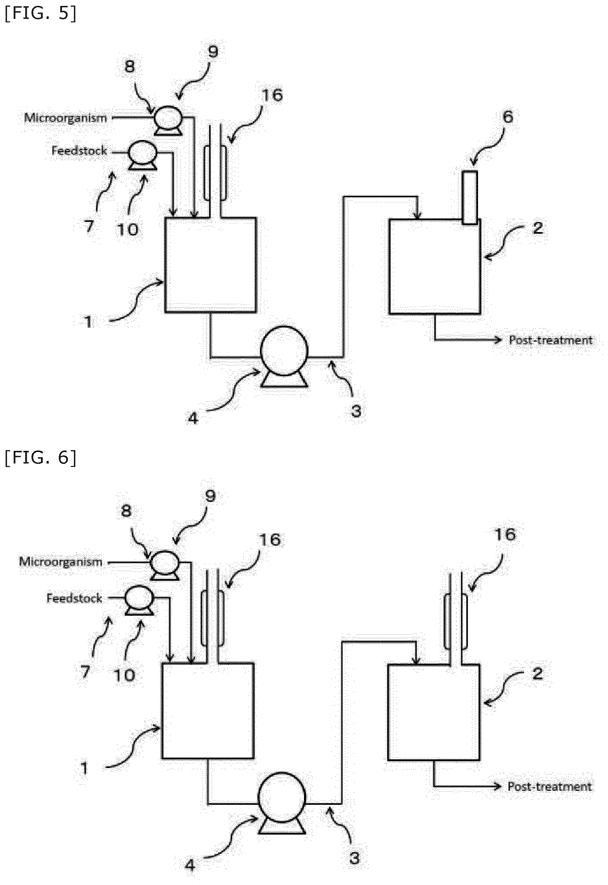

A test was carried out using the apparatus in FIG. 5. The apparatus in FIG. 5 was prepared by removing the gas connection pipe 5 from the apparatus in FIG. 4, and providing a vent 6 for the storage tank 2. Except for this, the test was carried out under the same conditions, and by the same operation, as in Example 1. The results are shown in FIG. 10. In Comparative Example 1, the ethanol concentration in the storage tank decreased with time.

(Comparative Example 2)

A test was carried out using the apparatus in FIG. 6. The apparatus in FIG. 6 is the same as the apparatus in FIG. 5 except that the apparatus in FIG. 6 comprises a backflow condenser instead of the vent 6 for the storage tank. Except for this, the test was carried out under the same conditions, and by the same operation, as in Example 1. The results are shown in FIG. 10.

In Comparative Example 2, the decrease in the ethanol concentration in the storage tank was improved compared to Comparative Example 1 because of the placement of the backflow condenser for the storage tank. However, similarly to Comparative Example 1, the ethanol concentration in the storage tank decreased with time, indicating a lower improving effect compared to the result in Example 1.

Thus, based on the results in Example 1 and Comparative Examples 1 and 2, it was found that, by connecting the gas phase portion in the culture tank and the gas phase portion in the storage tank to each other through a gas connection pipe, discharge of a volatile chemical substance can be prevented, thereby achieving a higher discharge-suppressing effect than that in the method of Comparative Example 2, which uses a backflow condenser.

(Example 2)

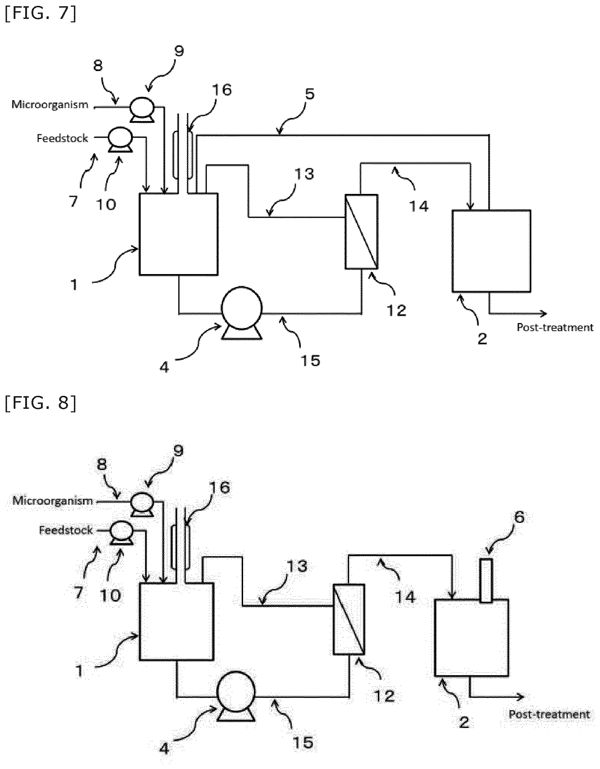

A test was carried out using the culture apparatus illustrated in FIG. 7. Since carbon dioxide is generated during the ethanol fermentation, a backflow condenser 16 was placed for the culture tank 1. The molasses prepared in Reference Example 3 was used as a feedstock. Culture and inoculation of the yeast were carried out similarly to Example 1, and immediately thereafter, a culture liquid circulation pump was operated to perform liquid circulation between the separation membrane module and the culture tank for 24 hours. Thereafter, filtration of the filtrate from the separation membrane module was started. After the beginning of the filtration, continuous ethanol fermentation was carried out for 600 hours under the following continuous fermentation conditions while carrying out control of addition of the fermentation feedstock such that the culture liquid volume in the continuous fermentation apparatus was 700 ml. Through the delivery line 15 and the filtrate pipe 14, the filtrate was delivered to the storage tank 2, which is connected to the culture tank 1 through the gas connection pipe 5. The filtrate was then retained therein. Changes in the ethanol concentration in the storage tank, during the steady state of the continuous culture, were measured over time by the method in Reference Example 1 from Hour 200 after the beginning of the inoculation. Between Hour 200 and Hour 600 after the beginning of the culture, the average ethanol concentration was 64.1 g/l.

[Continuous Fermentation Conditions]

For the continuous fermentation using the continuous fermentation apparatus 1, the following conditions were used.

Microorganism for the fermentation: *Schizosaccharomyces pombe* NBRC1628 strain
Amount of the fermentation microorganism inoculated: 10 v/v% (culture liquid volume [ml] / volume [ml])
Fermentation Conditions:
Fermentation reaction vessel capacity: 2 (l)
Separation membrane used: polyvinylidene fluoride filtration membrane
Effective filtration area of the separation membrane element: 218 ($cm^2$)
Temperature adjustment: 30 (°C)
Aeration rate in the fermentation reaction vessel: no aeration
Stirring rate in the fermentation reaction vessel: 300 (rpm) pH adjustment: none
Filtration flux set value (which is the same as the fermentation feedstock rate): 0.1 ($m^3/m^2$/day)
Sterilization: the separation membrane element and the fermentation tank were autoclaved at 121°C for 20 min
Average pore size: 0.1 $\mu$m
Standard deviation of the average pore size: 0.035 $\mu$m
Membrane surface roughness: 0.06 $\mu$m
Pure water permeability coefficient: $50 \times 10^{-9}$ $m^3/m^2$/s/pa

(Comparative Example 3)

A test was carried out using the apparatus in FIG. 8. The apparatus in FIG. 8 is the same as the apparatus in FIG. 7 except that the apparatus in FIG. 8 comprises a vent 6 instead of the gas connection pipe 5 for the storage tank 2. Except for this, the test was carried out under the same conditions as in Example 2. The average ethanol concentration between Hour 200 and Hour 600 was 57.0 g/l.

(Comparative Example 4)

A test was carried out using the apparatus in FIG. 9. The apparatus in FIG. 9 is the same as the apparatus in FIG. 8 except that the apparatus in FIG. 9 comprises a backflow condenser 16 for the storage tank 2 instead of the gas connection pipe 5 in FIG. 7. Except for this, the test was carried out under the same conditions, and by the same operation, as in Example 2. The average ethanol concentration between Hour 200 and Hour 600 was 60.7 g/l.

In Comparative Example 4, the decrease in the ethanol concentration in the storage tank was improved compared to Comparative Example 3 because of the placement of the backflow condenser for the storage tank. However, the average ethanol concentration was lower than that in the result of Example 2.

Thus, based on the results in Example 2 and Comparative Examples 3 and 4, it was found that, by connecting the gas phase portion in the culture tank and the gas phase portion in the storage tank to each other through a gas connection pipe, discharge of a volatile chemical substance can be prevented, thereby achieving a higher discharge-suppressing effect than that in the method of Comparative Example 4, which uses a backflow condenser.

[Explanation of References]

| | |
|---|---|
| 1, 1a, 1b | Culture tank |
| 2 | Storage tank |
| 3, 3a, 3b | Delivery line |
| 4, 4a, 4b | Pump |
| 5 | Gas connection pipe |
| 6 | Vent |
| 7 | Feedstock supply line |
| 8 | Microorganism supply line |
| 9 | Pump for feedstock supply line |
| 10 | Pump for microorganism supply line |
| 11, 11a | Multi-stage delivery line |
| 12 | Separation membrane module |
| 13 | Unfiltered-liquid pipe |
| 14 | Filtrate pipe |
| 15 | Culture liquid delivery line |
| 16 | Backflow condenser |

**Claims**

1. A method of producing a volatile chemical substance using a culture apparatus comprising: a culture tank configured to culture a microorganism having an ability to produce a volatile chemical substance; a storage tank configured to store a solution containing the chemical substance obtained by culturing the microorganism; and a delivery line configured to deliver a culture liquid obtained by culturing in the culture tank to the storage tank,
the method comprising culturing the microorganism in the culture tank while retaining the solution in the storage tank, wherein a gas phase portion in the storage tank and a gas phase portion in the culture tank are connected to each other through a gas connection pipe.

2. The method of producing a volatile chemical substance according to claim 1,
wherein the delivery line comprises: a separation membrane module configured to separate microorganisms in the culture liquid; a culture liquid delivery line configured to deliver the culture liquid to the separation membrane module; a filtrate pipe configured to deliver a filtrate obtained by the separation membrane module to the storage tank; and an unfiltered-liquid pipe configured to return an unfiltered liquid obtained by the separation membrane module to the culture tank, and
wherein in the delivery of the culture liquid from the culture tank to the storage tank, a filtrate obtained by passing the culture liquid through the separation membrane module is delivered to the storage tank as a solution containing the volatile chemical substance, and an unfiltered liquid obtained by passing the culture liquid through the separation membrane module is returned to the culture tank.

3. The method of producing a volatile chemical substance according to claim 2, wherein the separation membrane has an average pore size of 0.01 μm or more and less than 5 μm.

4. The method of producing a volatile chemical substance according to claim 2 or 3, wherein the separation membrane has a transmembrane pressure of 0.1 kPa or more and 150 kPa or less.

5.  The method of producing a volatile chemical substance according to any one of claims 1 to 4, wherein the liquid temperature in the culture tank is higher than the liquid temperature in the storage tank.

6.  The method of producing a volatile chemical substance according to any one of claims 1 to 5, wherein the vapor pressure of the volatile chemical substance is 1 kPa or more and 101 kPa or less at 30°C.

7.  The method of producing a volatile chemical substance according to any one of claims 1 to 6, wherein the volatile chemical substance is ethanol, methanol, 1-propanol, 2-propanol, 1-butanol, 2-methyl-1-propanol, 2-butanol, 2-methyl-2-propanol, acetone, or acetic acid.

8.  An apparatus for producing a volatile chemical substance, the apparatus comprising: a culture tank configured to culture a microorganism having an ability to produce a volatile chemical substance; a storage tank configured to store a solution containing the chemical substance obtained by culturing the microorganism; a delivery line configured to deliver a culture liquid obtained by culturing in the culture tank to the storage tank; and a gas connection pipe configured to connect a gas phase portion in the culture tank and a gas phase portion in the storage tank to each other.

9.  The apparatus for producing a volatile chemical substance according to claim 8, wherein the delivery line comprises: a separation membrane module configured to separate microorganisms in the culture liquid; a culture liquid delivery line configured to deliver the culture liquid to the separation membrane module; a filtrate pipe configured to deliver a filtrate obtained by the separation membrane module to the storage tank as a solution containing the volatile chemical substance; and an unfiltered-liquid pipe configured to return an unfiltered liquid obtained by the separation membrane module to the culture tank.

[FIG. 1]

[FIG. 2]

[FIG. 3]

[FIG. 4]

[FIG. 5]

[FIG. 6]

[FIG. 7]

[FIG. 8]

[FIG. 9]

[FIG. 10]

◆ : Ethanol concentration in storage tank in Example 1
△ : Ethanol concentration in storage tank in Comparative Example 1
□ : Ethanol concentration in storage tank in Comparative Example 2

## INTERNATIONAL SEARCH REPORT

| International application No. |
|---|
| PCT/JP2019/021450 |

**A.  CLASSIFICATION OF SUBJECT MATTER**
Int.Cl.  C12P7/04(2006.01)i, C12M1/00(2006.01)i

According to International Patent Classification (IPC) or to both national classification and IPC

**B.  FIELDS SEARCHED**

Minimum documentation searched (classification system followed by classification symbols)
Int.Cl.  C12P7/04, C12M1/00

Documentation searched other than minimum documentation to the extent that such documents are included in the fields searched
 Published examined utility model applications of Japan     1922–1996
 Published unexamined utility model applications of Japan   1971–2019
 Registered utility model specifications of Japan           1996–2019
 Published registered utility model applications of Japan   1994–2019

Electronic data base consulted during the international search (name of data base and, where practicable, search terms used)
 JSTPlus/JMEDPlus/JST7580(JDreamIII), CAplus/MEDLINE/EMBASE/BIOSIS/WPIDS(STN)

**C.  DOCUMENTS CONSIDERED TO BE RELEVANT**

| Category* | Citation of document, with indication, where appropriate, of the relevant passages | Relevant to claim No. |
|---|---|---|
| A | WO 2017/175833 A1 (CHUETSU-PULP AND PAPER CO., LTD.) 12 October 2017, entire text (Family: none) | 1–9 |
| A | JP 2011-530304 A (STATOIL ASA) 22 December 2011, entire text & US 2011/0171705 A1 & GB 2462642 A & WO 2010/018387 A2 & EP 2321420 A2 & CN 102186981 A | 1–9 |
| A | US 2011/0318800 A1 (CHEVRON U.S.A. INC.) 29 December 2011, entire text (Family: none) | 1–9 |

☒  Further documents are listed in the continuation of Box C.　　　☐  See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 25.06.2019 | 09.07.2019 |

| Name and mailing address of the ISA/ | Authorized officer |
|---|---|
| Japan Patent Office 3-4-3, Kasumigaseki, Chiyoda-ku, Tokyo 100-8915, Japan | Telephone No. |

Form PCT/ISA/210 (second sheet) (January 2015)

**INTERNATIONAL SEARCH REPORT**

| International application No. |
| --- |
| PCT/JP2019/021450 |

C (Continuation).  DOCUMENTS CONSIDERED TO BE RELEVANT

| Category* | Citation of document, with indication, where appropriate, of the relevant passages | Relevant to claim No. |
| --- | --- | --- |
| A | JP 2010-246512 A (BIO TRUST KK) 04 November 2010, entire text<br>(Family: none) | 1-9 |
| A | JP 2013-59274 A (HONDA MOTOR CO., LTD.) 04 April 2013, entire text<br>(Family: none) | 1-9 |

Form PCT/ISA/210 (continuation of second sheet) (January 2015)

**EP 3 805 398 A1**

**Patent documents cited in the description**

- JP 2010246512 A
- JP 2005013048 A
- WO 2007097260 A